# EUROPEAN PATENT APPLICATION

(11) **EP 4 343 308 A1**
(43) Date of publication of application: **27.03.2024**
(21) Application number: 22804457.4
(22) Date of filing: 06.04.2022
(51) Int. Cl.: G01N 21/27, G01F 23/292

(54) **BIOLOGICAL SAMPLE MEASUREMENT DEVICE**

(30) Priority: 21.05.2021 JP 2021085829
(71) Applicant: HITACHI HIGH-TECH CORPORATION, Tokyo 105-6409 (JP)
(72) Inventor: SAO, Mayu, Tokyo 100-8280 (JP); LEE,Yong, Tokyo 100-8280 (JP); KAWAMURA, Tomoto, Tokyo 100-8280 (JP); KAKUNO, Masahito, Tokyo 105-6409 (JP); SATO, Fumiyasu, Tokyo 105-6409 (JP)
(74) Representative: MERH-IP Matias Erny Reichl Hoffmann Patentanwälte PartG mbB
(86) International application number: PCT/JP2022/017188
(87) International publication number: WO 2022/244541

(57) **Abstract**

An object of the present invention is to provide a technique capable of accurately specifying a target portion as a measurement target without using a large imaging mechanism when measuring a biological sample separated into a plurality of component regions. A biological sample measurement device according to the present disclosure generates a captured image of a biological sample by using two wavelength components emitted from a light source, and compares the captured images generated by using two or more exposure times or gains at the time of imaging to select the captured image that can most clearly identify a measurement target portion in the biological sample (see FIG. 1).

## Description

### Technical Field

The present disclosure relates to a device for measuring a biological sample separated into a plurality of component regions.

### Background Art

For the purpose of improving an efficiency of clinical examinations such as blood examinations, there has been a demand for a technique for automating a liquid amount confirmation operation of a biological specimen before opening (before pipetting), which has been conventionally performed by visual confirmation. In particular, a biological sample such as a blood specimen before pipetting is configured to be separated into a plurality of layers by centrifugation or the like, and there is a need for a technique of measuring only a liquid amount of a sample to be analyzed among them.

As such a biological sample measurement device, for example, PTL 1 discloses a technique of irradiating a sample separated into a plurality of layers with pulsed light of two wavelengths in a time-division manner, and measuring transmitted light while scanning the sample in a vertical direction, thereby detecting a height of a predetermined region of the sample separated into the plurality of layers.

PTL 2 discloses a liquid detection device that applies infrared light to a specimen, detects transmitted light with a line sensor, obtains a boundary of a liquid layer based on a primary differential value of the transmitted light, and measures a serum amount of the specimen.

### Citation List

### Patent Literatures

PTL 1: US2012/0013889
PTL 2: JP 2004-37322 A

### Summary of Invention

### Technical Problem

The technique disclosed in PTL 1 relates to a liquid amount measurement technique for measuring a liquid amount by accurately specifying a boundary of a measurement target region from signals of transmitted light of two wavelengths having different absorption rates with respect to a measurement target for a biological sample including a plurality of components. However, in PTL 1, it is necessary to perform measurement while vertically scanning a specimen (blood collection tube) in a long axis direction, and there is a problem that it is difficult to downsize a device by a scanning mechanism.

In the technique described in PTL 2, an upper surface and a lower surface of serum are detected by signal processing based on differentiation from a signal of infrared transmitted light, and a serum amount is measured. However, when infrared rays are absorbed or scattered by characters printed on a blood collection tube, a blood cell component in a separation agent, color characters of a label, and the like, and transmitted light is attenuated, it is difficult to separate these noises from a boundary of serum, and there is a problem that accuracy of serum boundary specification and liquid amount measurement is reduced.

The present disclosure has been made in view of the above problems, and an object thereof is to provide a technique capable of accurately specifying a target portion as a measurement target without using a large imaging mechanism when measuring a biological sample separated into a plurality of component regions.

### Solution to Problem

A biological sample measurement device according to the present disclosure generates a captured image of a biological sample by using two wavelength components emitted from a light source, and compares the captured images generated by using two or more exposure times or gains at the time of imaging to select the captured image that can most clearly identify a measurement target portion in the biological sample.

### Advantageous Effects of Invention

According to the biological sample measurement device of the present disclosure, when a biological sample separated into a plurality of component regions is measured, a target portion as a measurement target can be accurately specified without using a large imaging mechanism.

### Brief Description of Drawings

[FIG. 1] FIG. 1 is a configuration diagram of a biological sample measurement device 100 according to a first embodiment.
[FIG. 2A] FIG. 2A is a diagram for describing a principle of specifying a measurement target region 109.
[FIG. 2B] FIG. 2B is a diagram for describing a principle of specifying the measurement target region 109.
[FIG. 3A] FIG. 3A illustrates an example in which a label is attached to a container of a biological sample 101.
[FIG. 3B] FIG. 3B illustrates an example in which a label is attached to the container of the biological sample 101.
[FIG. 4] FIG. 4 is a flowchart illustrating a procedure in which a time division control driver 104 sets a plurality of optical wavelengths and a plurality of exposure times.
[FIG. 5] FIG. 5 is a flowchart for describing an operation of an image processing unit 105.
[FIG. 6] FIG. 6 is a flowchart for describing details of S502.
[FIG. 7] FIG. 7 illustrates an example of processing of specifying the measurement target region 109.
[FIG. 8] FIG. 8 is a flowchart for describing details of S503.
[FIG. 9] FIG. 9 is a flowchart illustrating another procedure in which the time division control driver 104 sets a plurality of optical wavelengths and a plurality of exposure times.
[FIG. 10] FIG. 10 is a flowchart for describing an operation of a liquid amount calculation unit 108.
[FIG. 11] FIG. 11 is a configuration diagram of a biological sample measurement device 100 according to a second embodiment.
[FIG. 12] FIG. 12 is a configuration diagram of a biological sample measurement device 100 according to a third embodiment.

### Description of Embodiments

### <First Embodiment>

A biological sample to be measured in the first embodiment of the present disclosure is assumed to be a specimen before opening (before analysis) and separated into a plurality of component layers (typically, one to three layers) depending on a presence or absence of centrifugation. A measurement target is, for example, a portion such as plasma or serum among specimens separated into a plurality of layers.

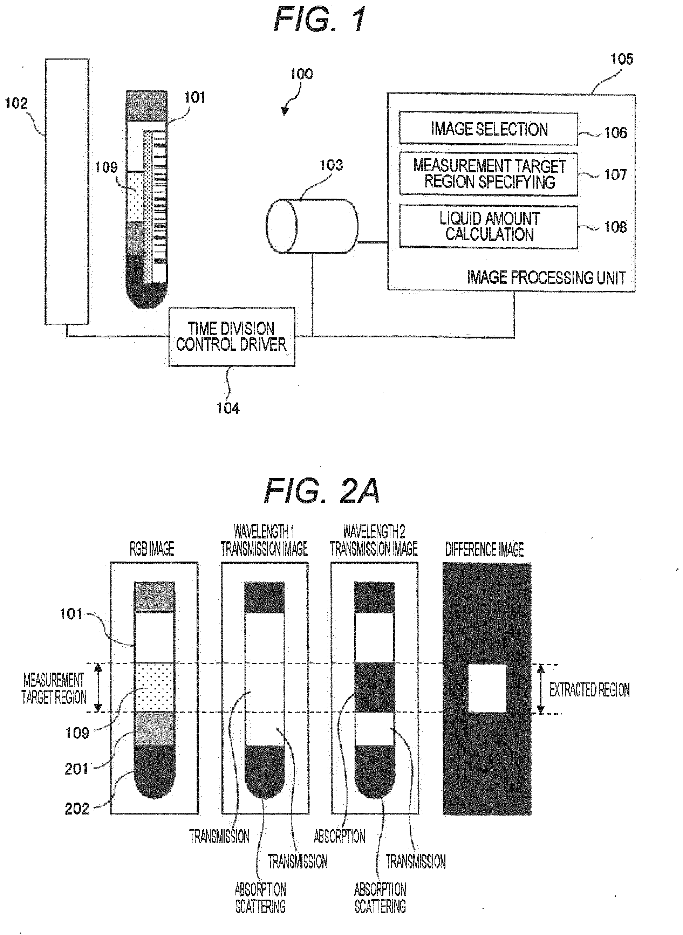

FIG. 1 is a configuration diagram of a biological sample measurement device 100 according to the first embodiment. The biological sample measurement device 100 is a device that measures a biological sample 101. The biological sample 101 is a sample configured as described above. The biological sample measurement device 100 specifies a measurement target region 109 of the biological sample 101 and measures a liquid amount and components thereof. The biological sample measurement device 100 includes a surface illumination light source 102, an area camera 103, a time division control driver 104, and an image processing unit 105.

The surface illumination light source 102 is configured to be able to switch a wavelength of emitted light between two wavelengths, and illuminates the biological sample 101 with the light. The light emitted from the surface illumination light source 102 can illuminate two or more component layers constituting the biological sample 101 at the same time (that is, across two or more component layers); The switching of the wavelengths of light does not necessarily mean emitting light having only a single wavelength, and it is sufficient that a wavelength component having a strongest intensity can be switched.

The area camera 103 generates a two-dimensional captured image of the biological sample 101 by imaging surface illumination light transmitted through the biological sample 101. The area camera 103 has sensitivity characteristics capable of detecting light in a wavelength band emitted from the surface illumination light source 102. The area camera 103 can be configured by, for example, an InGaAs camera or the like.

The time division control driver 104 switches the wavelength of the light emitted from the surface illumination light source 102 in a time-division manner. The time division control driver 104 adjusts an exposure time (or gain) of the area camera 103 to a time suitable for the wavelength in synchronization with the wavelength switching. The imaging timing by the area camera 103 is controlled by the time division control driver 104 in synchronization with the wavelength emitted by the surface illumination light source 102. The time division control driver 104 may receive a processing result from the image processing unit 105 and control re-imaging according to the result.

The image processing unit 105 extracts the measurement target region 109 from the captured image of each wavelength acquired by the area camera 103. The image processing unit 105 selects an optimum analysis image among the captured images having different exposure times, and calculates a liquid amount of the measurement target region 109 by using the captured image. The plurality of captured images having different exposure times can be acquired by the time division control driver 104 controlling the surface illumination light source 102 and the area camera 103.

The image processing unit 105 includes an image selection unit 106, a measurement target region specifying unit 107, and a liquid amount calculation unit 108. The image selection unit 106 selects an optimum image suitable as an analysis target from images imaged by the area camera 103. The measurement target region specifying unit 107 specifies the measurement target region 109 by comparing the captured images of the respective wavelengths. The liquid amount calculation unit 108 calculates a liquid amount of the measurement target region 109. These detailed operations will be described later.

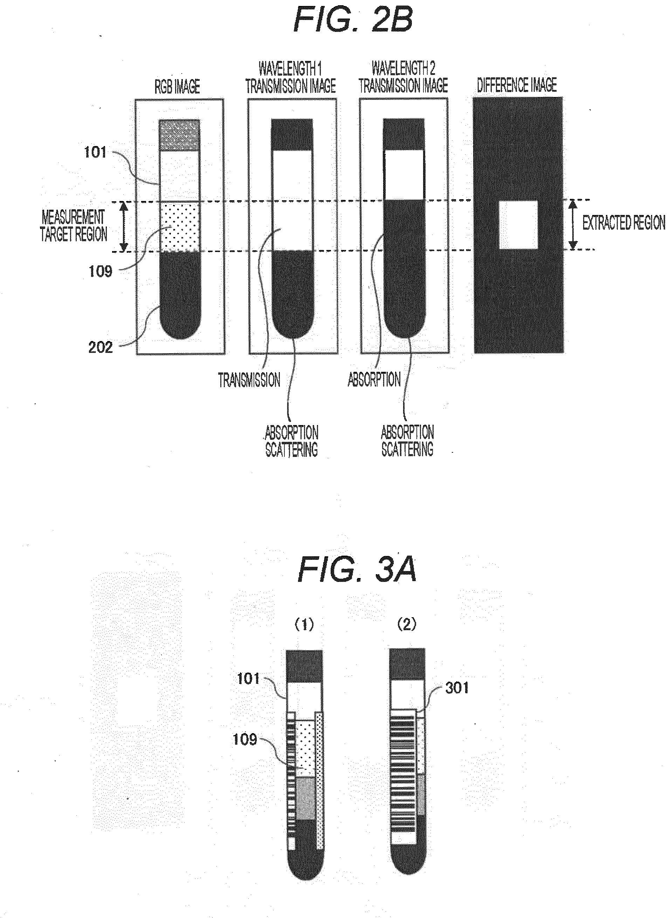

FIGS. 2A and 2B are diagrams for describing a principle of specifying the measurement target region 109. FIG. 2A illustrates an example in which the biological sample 101 is separated into three layers. FIG. 2B illustrates an example in which the biological sample 101 is separated into two layers. A clot 202 is formed in a lower layer when the biological sample 101 is centrifuged. A separation agent 201 is mixed to separate the clot 202 and the measurement target region 109.

Comparing a first wavelength (wavelength 1) and a second wavelength (wavelength 2) emitted from the surface illumination light source 102, a transmittance when the wavelength 1 is transmitted through the clot 202 is substantially the same as a transmittance when the wavelength 2 is transmitted through the clot 202. Therefore, a difference between the captured image of the clot 202 acquired by using the wavelength 1 and the captured image of the clot 202 acquired by using the wavelength 2 is very small. Similarly, regarding the separation agent 201, the transmittance at the wavelength 1 and the transmittance at the wavelength 2 are substantially the same, and thus the difference between the two images is very small.

On the other hand, the transmittance when the wavelength 1 is transmitted through measurement target region 109 is greatly different from the transmittance when the wavelength 2 is transmitted through measurement target region 109. Therefore, the difference between the captured image of the measurement target region 109 acquired by using the wavelength 1 and the captured image of the measurement target region 109 acquired by using the wavelength 2 is remarkable. By identifying this difference, the measurement target region 109 can be extracted from the captured image.

As illustrated in FIGS. 2A and 2B, the wavelength bands adopted as the wavelength 1 and the wavelength 2 need to be selected in advance such that a remarkable difference occurs between the wavelengths in the measurement target region 109, but no difference occurs in other portions. A specific numerical value of the wavelength may be arbitrary as long as this condition is satisfied. That is, at least a difference between the transmittance when the wavelength 1 is transmitted through the measurement target region 109 and the transmittance when the wavelength 2 is transmitted through the measurement target region 109 may be larger than a difference between the transmittance when the wavelength 1 is transmitted through a region other than measurement target region 109 and the transmittance when the wavelength 2 is transmitted through a region other than measurement target region 109.

By using the above measurement principle, when the biological sample 101 is separated into a plurality of component layers, the measurement target region 109 can be specified. The measurement target region specifying unit 107 specifies the measurement target region 109 according to this principle. The number of component layers is not limited, but in a typical biological sample such as plasma or serum, the component layers are separated into one to three layers. In either case, the measurement target region 109 can be specified with high accuracy.

There is a case where a label is attached to a container of the biological sample 101. In this case, the transmitted light is attenuated by the label. However, since the transmitted light is attenuated at both the wavelength 1 and the wavelength 2, when there is attenuation at both the wavelengths, it can be estimated that the label is attached to the portion. That is, it is possible to distinguish between attenuation by the label and attenuation by the measurement target region 109.

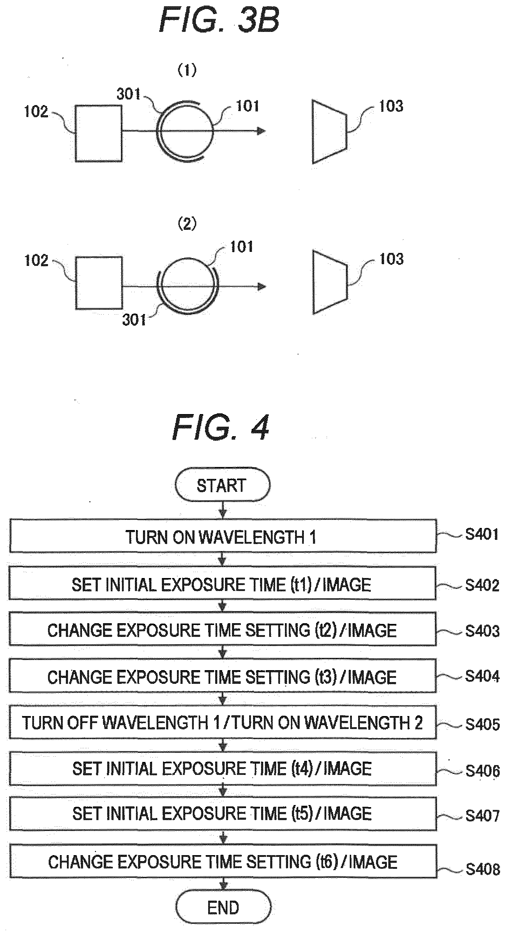

FIGS. 3A and 3B illustrate an example in which a label is attached to a container of the biological sample 101. FIG. 3A is a side view of the container, and FIG. 3B is a top view thereof. A barcode label, a pre-label, or the like maybe attached to the container (for example, a blood collection tube) of the biological sample 101. Even in such a case, it is required to accurately specify the measurement target region 109.

As illustrated in (1) of FIG. 3B, when a case where the light from the surface illumination light source 102 is transmitted through a label 301 once is compared with a case where the light is transmitted through the label 301 twice as illustrated in (2) of FIG. 3B, the latter has larger attenuation of the light. When the attenuation is large, a contrast difference between the measurement target region 109 and the other regions decreases, and this also reduces the calculation accuracy of the liquid amount.

Therefore, in the first embodiment, by adjusting the exposure time (or gain, the same applies hereinafter) of the area camera 103, it is possible to obtain an optimum captured image regardless of a light attenuation amount. When the attenuation is large, the exposure time is lengthened (or the gain is increased). However, an optimum exposure time varies depending on an orientation of the biological sample 101 (the number of times the light passes through the label). For example, when the exposure time is optimized under a condition of (2) of FIG. 3B, the exposure time is too long and the transmitted light intensity is saturated under, a condition of (1) of FIG. 3B or no label. On the other hand, when the exposure time is optimized under the condition of (1) of FIG. 3B, the light amount is insufficient in (2) of FIG. 3B.

In view of the above, in the first embodiment, the captured image is acquired in each exposure time while changing the exposure time, and the image processing unit 105 compares the respective captured images to select the most suitable captured image for specifying the measurement target region 109. As a result, it is possible to obtain a captured image most suitable for calculating the liquid amount of the measurement target region 109 regardless of the orientation of the biological sample 101. Furthermore, a mechanism or the like for rotating the biological sample 101 is also unnecessary. A procedure for selecting an optimum image will be described later.

FIG. 4 is a flowchart illustrating a procedure in which the time division control driver 104 sets a plurality of optical wavelengths and a plurality of exposure times. The time division control driver 104 switches the wavelength of the light emitted from the surface illumination light source 102 between two wavelengths. Further, the time division control driver 104 sets a plurality of exposure times for each wavelength in order to specify an optimum exposure time for each wavelength. Each step of FIG. 4 will be described below.

### (FIG. 4: Steps S401 to S404)

The time division control driver 104 controls the surface illumination light source 102 to emit the wavelength 1 (S401). The time division control driver 104 increases the exposure time of the area camera 103 in the order of t1, t2, and t3 (t1 < t2 < t3), and acquires the captured image of the biological sample 101 by the area camera 103 at each exposure time (S402 to S404).

### (FIG. 4: Steps S405 to S408)

The time division control driver 104 controls the surface illumination light source 102 to stop the wavelength 1 and emit the wavelength 2 instead (S405). The time division control driver 104 increases the exposure time of the area camera 103 in the order of t4, t5, and t6 (t4 < t5 < t6), and acquires the captured image of the biological sample 101 by the area camera 103 at each exposure time (S406 to S408).

### (FIG. 4: Steps S406 to S408: Supplement)

t4 to t6 do not necessarily have the same time length as t1 to t3, respectively. However, in view of the acquisition of a difference image described in FIGS. 2A and 2B, it is desirable to set each exposure time such that the captured image acquired at the wavelength 1 and the captured image acquired at the wavelength 2 are generated with substantially the same light amount. For the same reason, for example, comparison between the exposure times t1 and t5 or comparison between the exposure times t1 and t6 is not desirable.

### (FIG. 4: Steps S402 to S404 and S406 to S408: Supplement)

In this flowchart, although an example in which three exposure times are used for each wavelength is shown, when at least two exposure times are used for each wavelength, it is possible to compare captured images obtained by the respective exposure times and select an optimum captured image.

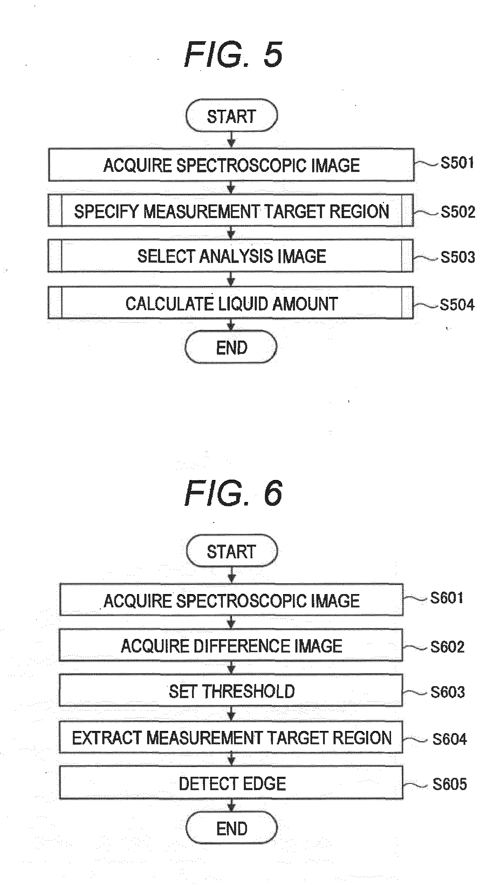

FIG. 5 is a flowchart for describing the operation of the image processing unit 105. The image processing unit 105 acquires captured images for each wavelength captured by the area camera 103 (S501), and specifies the measurement target region 109 by comparing these captured images (S502). The image processing unit 105 selects a captured image suitable for calculating the liquid amount by comparing the captured images acquired in the respective exposure times (S503). The image processing unit 105 calculates the liquid amount in the measurement target region 109 (S504). Details of each step will be described below.

FIG. 6 is a flowchart for describing details of S502. This flowchart is performed by the measurement target region specifying unit 107.

### (FIG. 6: Step S601)

The measurement target region specifying unit 107 acquires captured images at the wavelength 1 and the wavelength 2 respectively. In this flowchart, since it is sufficient to specify the measurement target region 109 according to the procedure described in FIGS. 2A and 2B, for example, an exposure time determined in advance as a specified value may be used.

### (FIG. 6: Step S602)

The measurement target region specifying unit 107 calculates a difference image between the captured image at the wavelength 1 and the captured image at the wavelength 2. In this step, the difference image described in FIGS. 2A and 2B is calculated, and the difference image can be acquired by calculating a difference between the pixel values of the respective images. Examples of the calculation procedure include: (a) image at wavelength 1 - image at wavelength 2; (b) image at wavelength 2 - image at wavelength 1; and (c) (image at wavelength 1 - image at wavelength 2)/(image at wavelength 1 + image at wavelength 2).

### (FIG. 6: Step S602: Supplement)

The combination of the image at the wavelength 1 and the image at the wavelength 2 is performed in the same exposure time. For example, captured images acquired respectively at the exposure time t1 for the wavelength 1 and at the exposure time t4 for the wavelength 2 are combined. The same applies to the exposure times t2 and t5, and t3 and t6. The difference image may be generated by using all the combinations of the exposure times, or only one combination capable of sufficiently specifying the measurement target region 109 on the difference image may be used.

### (FIG. 6: Step S603)

The measurement target region specifying unit 107 sets a threshold for detecting the measurement target region 109. The threshold can be set such that, for example, pixel value = 0, or an appropriate threshold may be set for each sample.

### (FIG. 6: Step S604)

The measurement target region specifying unit 107 extracts, as the measurement target region 109, a portion of the difference image in which the pixel value is equal to or larger than the threshold.

### (FIG. 6: Step S605)

The measurement target region specifying unit 107 detects an edge (height) of the measurement target region 109. As an edge detection method, for example, the following can be used: (a) an edge detection algorithm based on a luminance gradient (inclination) in the vertical direction, a differential value, or the like; and (b) the extracted pixel value of the measurement target region 109 is integrated in the horizontal direction to be one-dimensional, and the edge in the vertical direction is specified by using an inclination, a differential value, a variance value, and the like of the signal in the vertical direction. The specification result can be stored as a coordinate value on the captured image. That is, by calculating statistics of pixel values in the horizontal direction as features in the horizontal direction and comparing the features along the vertical direction, upper and lower edges of the measurement target region 109 can be specified.

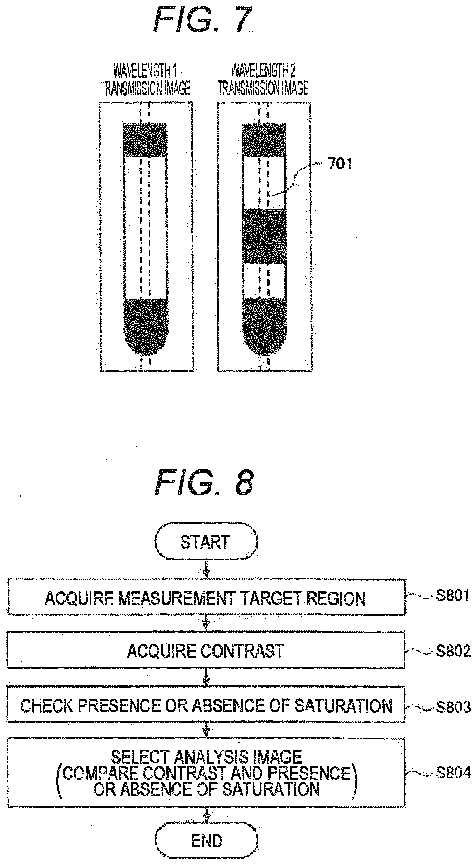

FIG. 7 illustrates an example of processing of specifying the measurement target region 109. The flowchart of FIG. 6 may be performed on a two-dimensional captured image as illustrated in FIG. 2, or may be performed by using a pixel value obtained by extracting a center region from the captured image and calculating an average value or a median value in the horizontal direction as illustrated in FIG. 7 to make the pixel value one-dimensional.

FIG. 8 is a flowchart for describing details of S503. This flowchart is performed by the image selection unit 106.

.

### (FIG. 8: Step S801)

The image selection unit 106 acquires a pixel value of the measurement target region 109.

### (FIG. 8: Step S802)

The image selection unit 106 calculates a contrast ratio between the measurement target region 109 and a portion adjacent thereto in the captured image of the biological sample 101 for each exposure time of the area camera 103. Specifically, a contrast ratio between each region adjacent above and below the measurement target region 109 and the measurement target region 109 is calculated.

### (FIG. 8: Step S802: Supplement)

In the flowchart of FIG. 4, since captured images are acquired by combinations of exposure times t1 and t4, t2 and t5, and t3 and t6 respectively, these captured images can be used in this step. For example, the contrast ratio in the captured image acquired at each of the exposure times t1, t2, and t3 may be obtained, or the contrast ratio in all the exposure times t1 to t6 may be obtained, Furthermore, the contrast ratio may be obtained by using a difference image (difference image output in step S602 in the flowchart of FIG. 6) acquired by combinations of the exposure times t1 and t4, t2 and t5, and t3 and t6.

### (FIG. 8: Step S803)

The image selection unit 106 checks whether the pixel value of the region adjacent to the measurement target region 109 is saturated for the captured image of each exposure time. For example, when the exposure time is too long, a luminance value may be saturated. Whether the pixel value of the measurement target region 109 itself is saturated may also be checked.

### (FIG. 8: Step S804)

The image selection unit 106 selects, as an image to be used for liquid amount analysis, a captured image in which the contrast ratio between the measurement target region 109 and the adjacent region thereof is the highest (that is, the measurement target region 109 can be most clearly identified) and the pixel value of the adjacent region is not saturated.

### (FIG. 8: Step S804: Supplement)

A captured image having the highest contrast ratio between the measurement target region 109 and the region adjacent to an upper side thereof may be selected, and a captured image having the highest contrast ratio between the measurement target region 109 and the region adjacent to a lower side thereof may be separately selected. This improves the accuracy of liquid amount calculation. Alternatively, the contrast ratio may be simply determined in only one of the upper adjacent region and the lower adjacent region.

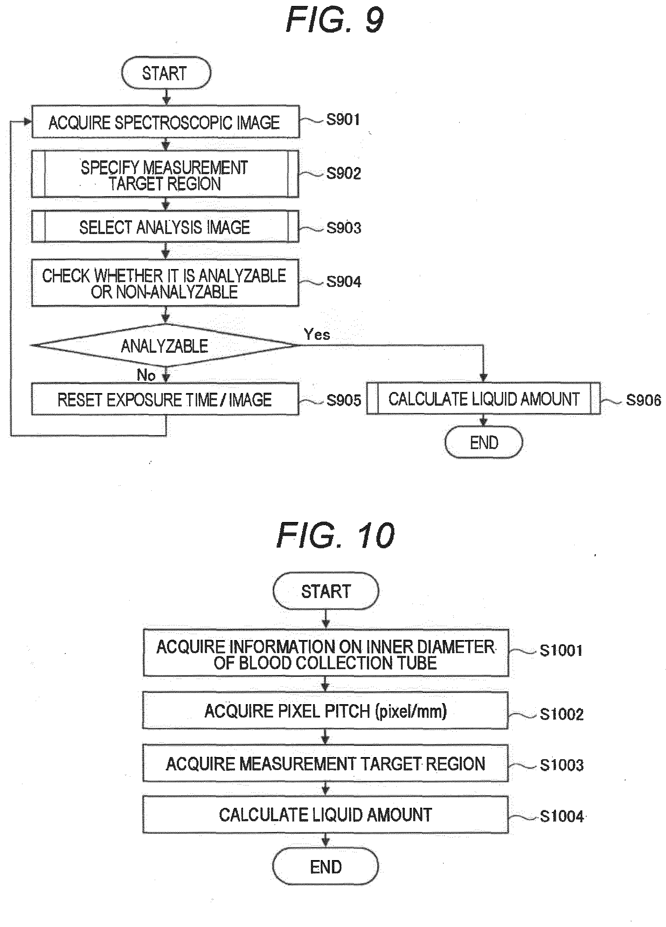

FIG. 9 is a flowchart illustrating another procedure in which the time division control driver 104 sets a plurality of optical wavelengths and a plurality of exposure times. In FIG. 4, imaging is performed with a plurality of exposure times set in advance, and an image most suitable for liquid amount analysis is selected from among the images in FIGS. 5 to 8. On the other hand, in this flowchart, every time the exposure time is set, the contrast ratio between the measurement target region 109 and the adjacent region thereof is calculated, and whether it is suitable for liquid amount analysis is checked.

### (FIG. 9: Step S901)

The image processing unit 105 acquires captured images at the wavelength 1 and the wavelength 2 respectively. When this step is performed first, an initial value determined in advance is used as the exposure time. When this step is performed for a second time or later, the exposure time reset in S905 is used.

### (FIG. 9: Step S902)

The measurement target region specifying unit 107 specifies the measurement target region 109 by using the difference image between the captured image at the wavelength 1 and the captured image at the wavelength 2 as in S602.

### (FIG. 9: Step S903)

The image selection unit 106 calculates the contrast ratio between the measurement target region 109 and the adjacent region thereof and further checks whether the luminance value is saturated in the adjacent region. Since these processing are similar to those in the flowchart of FIG. 8, this step is referred to as "analysis image selection" processing for convenience.

### (FIG. 9: Step S904)

The image processing unit 105 checks whether the contrast ratio calculated in S903 is equal to or larger than the threshold. When the contrast ratio equal to or larger than the threshold is obtained, the processing proceeds to S906, and when the contrast ratio is not obtained, the processing proceeds to S905.

### (FIG. 9: Step S905)

The image processing unit 105 resets the exposure time of the area camera 103 and reimages the image of the biological sample 101. The exposure time to be reset may be determined in advance, or the exposure time may be reset according to the contrast ratio calculated in S903. In a case where the exposure time is determined in advance, for example, the exposure time is increased each time this step is performed with the shortest exposure time as an initial value. When the exposure time is reset according to the contrast ratio, for example, how much the exposure time is increased or decreased is determined according to the difference between the calculated contrast ratio and the threshold.

### (FIG. 9: Step S906)

The liquid amount calculation unit 108 calculates a liquid amount of the measurement target region 109.

According to the flowchart of FIG. 9, unlike FIG. 4, the processing proceeds to S906 when it is determined that the measurement target region 109 can be sufficiently specified, and thus, it is not necessary to acquire the captured image for all the exposure times. Therefore, a measurement time can be shortened as compared with FIG. 4.

FIG. 10 is a flowchart for describing the operation of the liquid amount calculation unit 108. This flowchart describes details of S504 and S906.

### (FIG. 10: Step S1001)

The liquid amount calculation unit 108 acquires an inner diameter of a container (for example, a blood collection tube) of the biological sample 101. Since a shape and a size of the container are known in advance, a numerical value of the inner diameter may be stored in advance in the image processing unit 105.

### (FIG. 10: Step S1002)

The liquid amount calculation unit 108 acquires a pixel pitch (pixel/mm, that is, an actual size of one pixel on an image) of an image captured by the area camera 103. As a method of obtaining the pixel pitch, there are a method of acquiring the pixel pitch by calibration in advance and a method of obtaining the pixel pitch from a captured image of the biological sample 101. The calibration is performed by analyzing a pixel size of an image obtained by imaging an article whose actual size is known in advance. In the case of using the captured image of the biological sample 101, a horizontal width of the container is detected • from the image, and is associated with a diameter of the blood collection tube to calculate the pixel pitch.

### (FIG. 10: Steps S1003 and S1004)

The liquid amount calculation unit 108 acquires an image of the measurement target region 109 (S1003). The liquid amount calculation unit 108 calculates a liquid amount of the measurement target region 109 by using the pixel size and the pixel pitch of the measurement target region 109 (S1004).

### <First embodiment: Summary>

The biological sample measurement device 100 according to the first embodiment specifies the measurement target region 109 by using the two wavelength components emitted from the surface illumination light source 102 and compares the captured images obtained by using two or more exposure times, thereby selecting a captured image that can most clearly identify the measurement target region 109. By comparing the contrast ratio for each exposure time, even in a case where a label is attached to the biological sample 101, an optimum image can be obtained without rotating the biological sample 101. Furthermore, by using the surface illumination light source 102, a mechanism for scanning the illumination light in the vertical direction is unnecessary. Therefore, it is possible to accurately calculate the liquid amount of the measurement target region 109 while downsizing the device.

### <Second Embodiment>

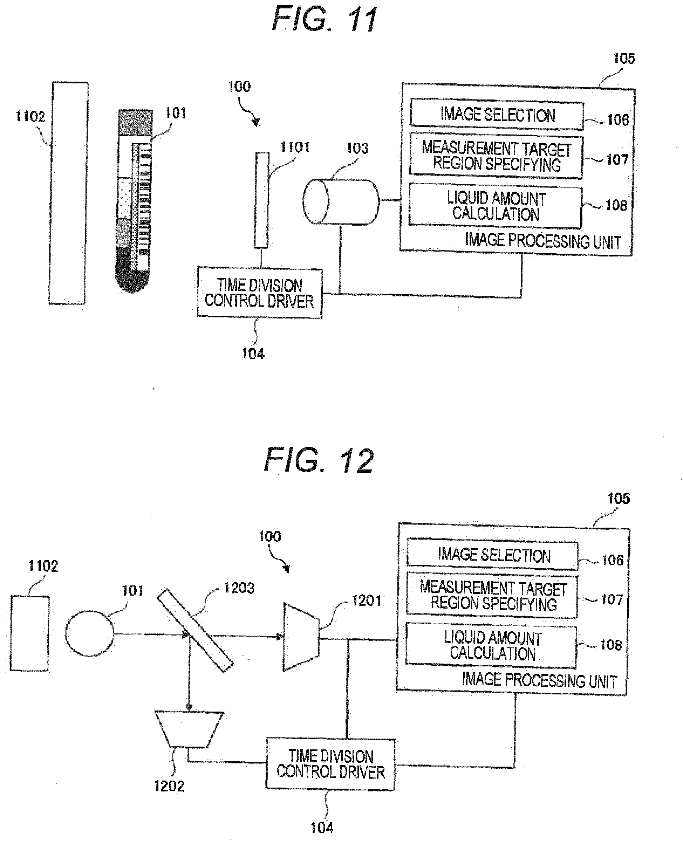

FIG. 11 is a configuration diagram of a biological sample measurement device 100 according to the second embodiment of the present disclosure. In the second embodiment, a surface illumination light source 1102 is used instead of the surface illumination light source 102. The surface illumination light source 1102 includes a white light source such as a halogen lamp or a two-wavelength light source that is turned on at the same time. In the present second embodiment, a bandpass filter 1101 is further provided. The bandpass filter 1101 allows light having a wavelength designated by the time division control driver 104 to pass. Therefore, the surface illumination light source 1102 does not need to switch the wavelength to be emitted, and instead, the time division control driver 104 switches a pass wavelength band of the bandpass filter 1101. As a result, similarly to the first embodiment, captured images of two wavelengths can be compared. The rest is the same as that in the first embodiment.

As a premise of the above, the surface illumination light source 1102 needs to emit light including two wavelengths having absorption rates different from each other in the measurement target region 109 (further, absorption rates do not greatly differ in a portion other than the measurement target region 109). The bandpass filter 1101 needs to be able to switch which of the two wavelengths is allowed to pass. An element other than the bandpass filter 1101 may be used as long as a similar function can be realized.

### <Third Embodiment>

FIG. 12 is a configuration diagram of a biological sample measurement device 100 according to the third embodiment of the present disclosure. In the third embodiment, an optical filter 1203 is provided instead of the bandpass filter 1101, and two area cameras 1201 and 1202 are provided instead of the area camera 103. The rest is the same as that in the second embodiment.

The optical filter 1203 transmits the wavelength 1 and reflects the wavelength 2. It is not always necessary to completely transmit the wavelength 1 and completely reflect the wavelength 2, and it is sufficient that a transmittance of the wavelength 1 is higher than a transmittance of the wavelength 2 and a reflectance of the wavelength 2 is higher than a reflectance of the wavelength 1. The area camera 1201 images an image of the light transmitted through the optical filter 1203, and the area camera 1202 images an image of the light reflected from the optical filter 1203. The time division control driver 104 adjusts the exposure time of each camera. In the third embodiment, since images of two wavelengths can be imaged at the same time, there is an advantage that the measurement time can be shortened.

### <Modifications of present invention>

The present invention is not limited to the above-described embodiments, and includes various modifications. For example, the above-described embodiments have been described in detail for easy understanding of the present invention, and are not necessarily limited to those having all the described configurations. Further, a part of a configuration of one embodiment can be replaced with a configuration of another embodiment, and the configuration of another embodiment can be added to the configuration of one embodiment. In addition, it is possible to add, delete, and replace other configurations for a part of the configuration of each embodiment.

In the above embodiment, the gain of the area camera 103 may be adjusted instead of or in combination with the exposure time of the area camera 103. In this case, in each flowchart, processing of increasing the gain may be performed instead of processing of increasing the exposure time.

The time division control driver 104 and the image processing unit 105 can be configured by hardware such as a circuit device implementing these functions, or can be configured by an arithmetic device executing software implementing these functions.

### Reference Signs List

- 100: biological sample measurement device
- 101: biological sample
- 102: surface illumination light source
- 103: area camera
- 104: time division control driver
- 105: image processing unit
- 1101: bandpass filter
- 1102: surface illumination light source
- 1201: area camera
- 1202: area camera
- 1203: optical filter

## Claims

1. A biological sample measurement device that measures a biological sample separated into a plurality of component regions, the biological sample measurement device comprising:
a light source configured to irradiate the biological sample with light;
an imaging device configured to generate a two-dimensional captured image of the biological sample by using the light transmitted through the biological sample; and
an image processing unit configured to specify a target portion as a measurement target in the component region from the captured image,
wherein the image processing unit specifies the target portion by using the captured image generated by using each of two wavelength components emitted by the light source,
the light source is configured to irradiate at least two of the component regions with the light at a same time,
the imaging device generates the captured image by using at least one of two or more exposure times or two or more gains, and
the image processing unit selects, as an image of the biological sample to be used for the measurement, the captured image that can most clearly identify a boundary between the target portion and other portions by comparing the captured images generated by using the two or more exposure times or the two or more gains.

2. The biological sample measurement device according to claim 1, wherein
the imaging device generates a first captured image of the biological sample by using a first wavelength component of the light transmitted through the biological sample,
the imaging device generates a second captured image of the biological sample by using a second wavelength component of the light transmitted through the biological sample,
the image processing unit calculates a difference between a portion generated by using the first wavelength component in the first captured image and a portion generated by using the second wavelength component in the second captured image, and
the image processing unit specifies a range of the target portion by specifying a portion in which the difference is equal to or larger than a threshold.

3. The biological sample measurement device according to claim 2, wherein a difference in a transmittance between the first wavelength component and the second wavelength component in the target portion is larger than a difference in a transmittance between the first wavelength component and the second wavelength component in a portion excluding the target portion.

4. The biological sample measurement device according to claim 2, wherein
the image processing unit calculates a feature value of the captured image in a first direction by performing statistical processing on pixel values of the captured image in the first direction, and
the image processing unit specifies a boundary of the target portion in a second direction by comparing the feature value along the second direction different from the first direction.

5. The biological sample measurement device according to claim 1, wherein
the image processing unit specifies a range of the target portion in the captured image,
the image processing unit specifies a boundary of the specified target portion according to a difference between the captured images, and
the image processing unit calculates an amount of the target portion by using the specified boundary.

6. The biological sample measurement device according to claim 1, wherein the image processing unit selects, as an image of the biological sample to be used for the measurement, the captured image in which a contrast between the target portion and other portions is highest and pixel values near the target portion are not saturated.

7. The biological sample measurement device according to claim 2, wherein
when generating the first captured image by using the first wavelength component, the imaging device generates the first captured image by using a first exposure time and a second exposure time longer than the first exposure time, or generates the first captured image by using each of a first gain and a second gain larger than the first gain,
when generating the second captured image by using the second wavelength component, the imaging device generates the second captured image by using a third exposure time and a fourth exposure time longer than the third exposure time, or generates the second captured image by using each of a third gain and a fourth gain larger than the third gain, and
the image processing unit selects the captured image in which the boundary can be most clearly identified by comparing the captured images generated by using the respective exposure times, or selects the captured image in which the boundary can be most clearly identified by comparing the captured images generated by using the respective gains.

8. The biological sample measurement device according to claim 1, wherein
the image processing unit determines whether the boundary has a contrast equal to or larger than a threshold in the captured image generated by the imaging device using a first exposure time or a first gain,
the imaging device regenerates the captured image by using a second exposure time longer than the first exposure time or a second gain larger than the first gain in a case where the boundary does not have the contrast equal to or larger than the threshold, and
the image processing unit redetermines whether the boundary has the contrast equal to or larger than the threshold in the regenerated captured image.

9. The biological sample measurement device according to claim 1, wherein the image processing unit calculates an amount of the target portion by using a pixel size of the captured image and a size of a container containing the biological sample.

10. The biological sample measurement device according to claim 2, wherein
the light source is configured to be able to switch a strongest main wavelength component of the light between at least the first wavelength component and the second wavelength component,
the biological sample measurement device further comprises a time division control driver that switches the main wavelength component in a time-division manner,
the imaging device generates the first captured image in a period in which the time division control driver sets the main wavelength component to the first wavelength component, and
the imaging device generates the second captured image in a period in which the time division control driver sets the main wavelength component to the second wavelength component.

11. The biological sample measurement device according to claim 2, wherein
the light source is configured to emit the light having the first wavelength component and the second wavelength component at a same time, and
the biological sample measurement device further comprises a filter capable of switching a wavelength component to be passed,
the imaging device generates the first captured image in a period in which the filter allows the first wavelength component to pass, and
the imaging device generates the second captured image in a period in which the filter allows the second wavelength component to pass.

12. The biological sample measurement device according to claim 2, wherein
the light source is configured to emit the light having the first wavelength component and the second wavelength component at a same time,
the biological sample measurement device further comprises an optical filter that allows the first wavelength component to pass therethrough and reflects the second wavelength component, and
the imaging device includes a first imaging device that images the first wavelength component that has passed through the optical filter, and a second imaging device that images the second wavelength component reflected from the optical filter.
